(19) Europäisches Patentamt
European Patent Office
Office européen des brevets

(11) Veröffentlichungsnummer: **0 262 532 B1**

(12) **EUROPÄISCHE PATENTSCHRIFT**

(45) Veröffentlichungstag der Patentschrift: **25.11.92**

(51) Int. Cl.5: **C07C 45/58**, C07C 49/04, C07C 47/228

(21) Anmeldenummer: **87113729.5**

(22) Anmeldetag: **19.09.87**

(54) **Verfahren zur Herstellung von Aldehyden und/oder Ketonen durch Umsetzung von Epoxiden.**

(30) Priorität: **25.09.86 DE 3632529**

(43) Veröffentlichungstag der Anmeldung:
**06.04.88 Patentblatt 88/14**

(45) Bekanntmachung des Hinweises auf die Patenterteilung:
**25.11.92 Patentblatt 92/48**

(84) Benannte Vertragsstaaten:
**BE CH DE FR GB LI NL**

(56) Entgegenhaltungen:
**EP-A- 0 100 117**
**US-A- 2 031 200**
**US-A- 2 660 609**
**US-A- 2 694 090**

**CHEMICAL ABSTRACTS, Band 103, Nr. 5, 5. August 1985, Zusammenfassung Nr. 36927s, Columbus, Ohio, US; E. RUIZ-HITZKY: "Epoxide rearrangements on mineral and silica-alumina surfaces"**

**PATENT ABSTRACTS OF JAPAN, Band 11, Nr. 192 (C-249)(1639), 19. Juni 1987; & JP-A-62 12736**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18**
**W-6710 Frankenthal(DE)**
Erfinder: **Goetz, Norbert, Dr.**
**Schoefferstrasse 25**
**W-6520 Worms 1(DE)**
Erfinder: **Hupfer, Leopold, Dr.**
**Waltershoehe 3**
**W-6701 Friedelsheim(DE)**
Erfinder: **Lermer, Helmut, Dr.**
**D 3,4**
**W-6800 Mannheim 1(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

PATENT ABSTRACTS OF JAPAN, Band 11, Nr. 193 (C-430)(2640), 20. Juni 1987; & JP-A-62 19549

METHODEN DER ORGANISCHEN CHEMIE, Band VII/2a, 4. Auflage, 1973, Seite 931-959; HOUBEN-WEYL, Georg Thieme Verlag Stuttgart

CHEMICAL ABSTRACTS, Band 89, Nr. 5, 31. Juli 1978, Zusammenfassung Nr. 42801e, Columbus, Ohio, USA; & JP-A-53 31637

## Beschreibung

Die Erfindung betrifft ein Verfahren zur Herstellung von Aldehyden und/oder Ketonen durch Umsetzung von Epoxiden an Zeolithen.

Verfahren zur Herstellung von Aldehyden und Ketonen sind in der Literatur mehrfach beschrieben. So sind verschiedene Verfahren bekannt, bei denen saure Homogenkatalysatoren wie Phosphorsäure, Bortrifluorid-Diethyletherat, Zinn-IV-Chlorid und Palladiumkomplexe für die Flüssigphasenreaktion verwendet werden. Für die Gasphasenreaktionen werden Aluminiumoxide und Aluminiumsilikate als Katalysatoren für die Umlagerung genannt.

Es ist auch bekannt, Butylenoxid an dotierten A-Zeolithen zu einem Gemisch aus Butyraldehyd, cis-trans-But-2-en-ol, Butanol und Methylethylketon umzusetzen. Doch ist die Selektivität bei dieser Reaktion noch ungenügend. Auch ist der A-Zeolith-Katalysator nach seiner Desaktivierung durch Koks nicht zu regenerieren, da bei den hierfür notwendigen Temperaturen von etwa 500 °C die Kristallstruktur des Zeolith zerstört wird. Dies gilt auch für die Herstellung von 2-(4')-Isobutyl-phenyl-propanal aus dem 2-4'-Isobutyl-phenyl-2-methyl-oxiran an 5 Å Molekularsieben (JP 3031-637).

Für die Umsetzung von Propylenoxid zu Aceton oder Propionaldehyd an alkalidotierten X-Zeolithen ist es notwendig in Abwesenheit stark acider Zentren zu arbeiten.

Cyclododecanon hat man an Pd- oder Rd-dot. $Al_2O_3$ aus Epoxycyclododecan erhalten, wobei darauf hingewiesen wird, daß Zeolithe für diese Reaktion ungeeignet sind (Neftekhimya 16 (1976) 250-254).

In EP-PS 100 117 wird die Reaktion von Styroloxid und von am aromatischen Kern alkyl- bzw. alkoxylsubstituierte Styroloxiden an einem Titan-haltigen Zeolithen zu $\beta$-Phenylaldehyden in der flüssigen Phase bei 30 bis 100 °C beschrieben. Der hierzu eingesetzte Katalysator wird aufwendig aus hochreinen Ausgangsstoffen wie Tetraalkylorthosilikat, Tetraalkylorthotitanat und Tetrapropylammoniumhydroxid hergestellt. Hohe Umsätze erzielt man nur bei Reaktion in einem Lösungsmittel wie Methanol oder Aceton bei 30 bis 100 °C in der Flüssigphase bei Verweilzeiten von 1 bis 1,5 h. Dies bedeutet erhöhten Aufwand für Destillation und Betrieb. An Titan-haltigen Zeolithen kann man nur Styroloxid und an Aromatenalkylierten bzw. alkoxylierten Styroloxiden umsetzen.

Es war die Aufgabe gestellt, ein Verfahren zu entwickeln, wonach auch bisher teilweise nicht zugängliche Aldehyde bzw. Ketone aus den entsprechenden Epoxiden in Gegenwart von einfach verfügbaren Katalysatoren hoher Aktivität und leichter Regenerierbarkeit hergestellt werden können. Weiterhin sollten bei langen Katalysatorstandzeiten hohe Umsätze und Selektivitäten und eine flexible Einsetzbarkeit des Katalysators hinsichtlich der Edukte erreicht werden.

Gegenstand der Erfindung ist ein Verfahren zur Herstellung von Aldehyden und Ketonen der Formel (I)

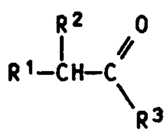

(I)

in der $R^1$ und $R^2$ gerade oder verzweigte Alkylreste mit einer C-Zahl von 1 bis 12, gerade oder verzweigte Alkenylreste mit einer C-Zahl von 1 bis 12, Cycloalkyl- oder Cycloalkenylreste mit 5 bis 8 Kohlenstoffatomen, Alkoxyreste oder Aryl- bzw. Aralkylreste, die ihrerseits substituiert sein können, bedeuten und $R^3$ ein Wasserstoff, Alkyl-, Alkenyl-, Aryl- oder Aralkylreste sein kann, dadurch gekennzeichnet, daß man Epoxide der Formel (II)

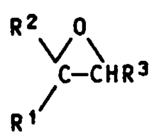

(II)

wobei $R^1$, $R^2$ und $R^3$ obige Bedeutung haben, unter Verwendung von einfach verfügbaren Katalysatoren hoher Aktivität und leichter Regenerierbarkeit umlagert.

Unter einfach verfügbaren Katalysatoren, hoher Aktivität und leichter Regenerierbarkeit im Sinne der Erfindung versteht man Zeolithe des Pentasil-Typs, des Mordenit-Typs, des Erionit-/Chabazit-Typs und des

L-Typs.

Durch die vorliegenden Erfindung wird ein Verfahren zur Herstellung der Aldehyde und Ketone der Formel (I) aus gut zugänglichen Ausgangsstoffen der Formel (II) in die Technik eingeführt, bei dem in Gegenwart von Katalysatoren, die sich durch einfache Verfügbarkeit, hohe Aktivität und leichte Regenerierbarkeit auszeichnen, bei langen Katalysatorstandzeiten, hohen Umsätzen und hohen Selektivitäten ein flexibler Einsatz der Katalysatoren hinsichtlich der Endprodukte gewährleistet ist.

Das erfindungsgemäße Verfahren vermeidet die eingangs erwähnten Nachteile der bekannten Verfahren. Das Ergebnis ist überraschend, da man bisher nur schwach acide X-Zeolithe als geeignet und Zeolithe für Umlagerungsreaktionen als ungeeignet einstufte. Daher konnte man nicht erwarten, daß gerade mit Zeolithen, die sich durch hohe Acidität sowie durch strenge Strukturparameter auszeichnen, in so weiten Grenzen und bei so großer Vielfalt der Edukte solch hervorragende Ergebnisse erhält.

Weitergehende Verteile des erfindungsgemäßen Verfahrens sind: Durch vollständigen Umsatz und Selektivität > 90 % ergeben sich auch keine Trennprobleme. Bei langen Standzeiten werden auch sehr gute Ausbeuten erreicht. Vorteilhaft sind auch die einfache Isolierung der Endprodukte, in der Regel Weiterverwendung ohne zusätzliche Reinigung, leichte Regenerierbarkeit der Katalysatoren bei eventuell auftretender Verkokung. Ein weiterer Vorteil ist, es, daß man die Reaktion in der Gasphase ausführen kann.

Für das erfindungsgemäße Verfahren können z.B. folgende Epoxide als Ausgangsstoffe verwendet werden: $\alpha$-Phenylstyroloxid (1,1-Diphenylethylenoxid), $\alpha$-Methylstyroloxid, $\alpha$-Ethylstyroloxid, $\alpha$-Isopropylstyroloxid, 2-Methyl-2,3-epoxibutan und Diisobutenoxid und andere.

Als Katalysatoren für die erfindungsgemäße Umwandlung von Epoxiden werden acide zeolithische Katalysatoren eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si-und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekeln besetzt.

In den Zeolithen können anstelle von Aluminium auch andere drei- und zweiwertige Elemente wie B, Ga, Fe, Cr, Be, As, Sb in das Gitter eingebaut werden oder das Silicium kann durch ein vierwertiges Element wie Ge, Ti, Zr, Hf, ersetzt werden.

Als Katalysatoren kommen Zeolithe aus der Mordenit-Gruppe oder Faujasit-Gruppe wie L-Zeolith oder engporige Zeolithe vom Erionit- bzw. Chabasit-Typ in Betracht. Besonders vorteilhaft für das erfindungsgemäße Verfahren sind Zeolithe vom Pentasil-Typ. Diese Zeolithe können unterschiedliche chemische Zusammensetzung aufweisen. Es handelt sich hierbei um Alumino-, Boro-, Eisen-, Gallium-, Chrom-, Beryllium-, Arsen-, Antimon- und Wismutsilikatzeolithe oder deren Gemische sowie um Alumino-, Boro-, Gallium- und Eisengermanatzeolithe oder deren Gemische.

Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in 1,6-Hexandiamin-oder 1,3-Propandiamin- oder Triethylentetramin-Lösung mit oder insbesondere ohne Alkali- oder Erdalkalizusatz bei 100 bis 220 °C unter autogenem Druck hergestellt. Geeignet sind auch die isotaktischen Zeolithe nach DE-OS 3 006 471. Die erhaltenen Aluminosilikatzeolithe weisen je nach Wahl der Einsatzstoffmenge ein $SiO_2/Al_2O_3$-Verhältnis von 10 bis 40.000 auf und können auch in etherischem Medium z.B. im Diethylenglykoldimethylether, in alkoholischen Medium wie Methanol bzw. 1,4-Butandiol oder in Wasser synthetisiert werden.

Die Borosilikatzeolithe und Einschluß der isotaktischen Borosilikatzeolithe können z.B. bis 90 bis 200 °C unter autogenem Druck synthetisiert werden, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit oder insbesondere ohne Alkali- oder Erdalkalizusatz zur Reaktion bringt. Die Borosilikatzeolithe können auch statt in wäßriger Aminlösung in etherischer Lösung, z.B. Diethylenglykoldimethylether oder in alkoholischer Lösung, z.B. 1,6-Hexandiol hergestellt werden.

Eisensilikatzeolithe erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Die so hergestellten Alumino-, Boro- und Eisensilikatzeolithe können nach ihrer Isolierung, Trockung bei 100 bis 160 °C, vorzugsweise 110 °C und Calcinierung bie 450 bis 550 °C, vorzugsweise 500 bis 540 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem

$SiO_2/Al_2O_3$-Verhältnis von 25:75 bis 95:5, bevorzugt 75:25, Siliciumdioxid, bevorzugt hochdisperses $SiO_2$, Gemische aus hochdispersem $SiO_2$ und hochdispersem $Al_2O_3$ sowie Ton. Nach der Verformung werden die Extrudate oder Preßlinge bei 110 ° C/16 h getrocknet und bei 500 ° C/16 h calciniert.

Man kann auch die isolierten Alumino- bzw. Borosilikatzeolithe direkt nach der Trocknung verformen und erstmals nach der Verformung einer Calcinierung unterwerfen. Die Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke. Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 ° C, bevorzugt 500 bis 540 ° C, zu regenerieren. Die Zeolithe erhalten dadurch ihre Anfangsaktivität zurück. Durch partielle Verkokung (pre-coke) ist es möglich, die Aktivität des Katalysators für ein Selektivitätsoptimum des gewünschten Reaktionsproduktes einzustellen.

Um eine möglichst große Selektivität, hohen Umsatz sowie lange Standzeiten zu erreichen, ist es mitunter vorteilhaft, die Zeolithe zu modifizieren. Eine geeignete Modifizierung der Katalysatoren besteht z.B darin, daß man die unverformten oder verformten Zeolithe mit Metallsalzen durch einen Ionenaustausch oder durch Imprägnierung dotiert.

Zweckmäßigerweise führt man die Dotierung so durch, daß man die verformten Zeolithe in einem Steigrohr vorlegt und bei 20 bis 100 ° C eine wäßrige oder ammoniakalische Lösung eines Halogenids oder eines Nitrats der Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf Zeolithe besteht darin, daß man das zeolithische Material z.B. mit einem Halogenid, einen Nitrat oder einem Oxid der Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht darin, daß man $Cs_2CO_3$ in Wasser löst. Mit dieser Lösung wird der verformte oder unverformte Zeolith eine gewisse Zeit, etwa 30 Minuten, getränkt. Die eventuell überstehende Lösung wird am Rotationsverdampfer von Wasser befreit. Danach wird der getränkte Zeolith bei etwa 150 ° C getrocknet und bei etwa 550 ° C calciniert. Dieser Tränkvorgang kann mehrmals hintereinander vorgenommen werden, um den gewünschten Metallgehalt einzustellen. Auch ist es möglich, eine ammoniakalische $Pd(NO_3)_2$-Lösung herzustellen und darin den reinen pulverförmigen Zeolithen bei 40 bis 100 ° C unter Rühren etwa 24 h aufzuschlämmen. Nach Abfiltrieren, Trocknen bei etwa 150 ° C und Calcinierung bei etwa 500 ° C kann das so gewonnene zeolithische Material mit oder ohne Bindemittel zu Strängen, Pellets oder Wirbelgut weiterverarbeitet werden. Ein Ionenaustausch des in der H-Form vorliegenden Zeolithen kann so vorgenommen werden, daß man den Zeolithen in Strängen oder Pellets in einer Kolonne vorlegt und darüber z.B. eine ammoniakalische $Pd(NO_3)_2$-Lösung bei leicht erhöhter Temperatur zwischen 30 und 60 ° C im Kreislauf 15 bis 20 h leitet. Danach wird mit Wasser ausgewaschen, bei etwa 150 ° C getrocknet und bei etwa 550 ° C calciniert. Bei manchen metalldotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft.

Zu den verwendbaren siliciumreichen Zeolithen ($SiO_2/Al_2O_3 \geqq 10$) gehören auch die bekannten ZSM-Typen sowie Ferrierit und Nu-1 sowie Silikalite ein Molekularsieb, ein sog. Silica Polymorph.

Die Katalysatoren können wahlweise als 2 bis 4 mm Stränge oder als Tabletten mit 3 bis 5 mm Durchmesser oder als Pulver mit Teilchengrößen von 0,1 bis 0,5 mm oder als Wirbelkontakt eingesetzt werden.

Die erfindungsgemäße Umwandlung der Epoxide wird bevorzugt in der Gasphase und in der Regel bei Temperaturen von 50 bis 500 ° C, vorzugsweise 150 bis 400 ° C, und einer Belastung WHSV = 0,1 bis 20 $h^{-1}$, vorzugsweise 0,5 bis 5 $h^{-1}$ (g Epoxid/g Katalysator und Stunde) ausgeführt. Die Reaktion kann im Festbett oder im Wirbelbett ausgeführt werden. Im allgemeinen steigt der Umsatz mit steigender Temperatur stark an, während die Selektivität in einem bestimmten Temperaturbereich nur wenig zurückgeht.

Es ist auch möglich, die Reaktion in der Flüssigphase (Suspensions-, Riesel- oder Sumpffahrweise) durchzuführen.

Das Verfahren wird im allgemeinen bei Normaldruck oder bei vermindertem oder erhöhtem Druck diskontinuierlich, vorzugsweise kontinuierlich durchgeführt.

Schwerflüchtige oder feste Ausgangsstoffe werden in gelöster Form z.B. in THF-, Toluol- oder Petrolether-Losung eingesetzt. Allgemein ist auch eine Verdünnung mit diesen Lösungsmitteln oder Inertgasen wie $N_2$, Ar, $H_2O$-Dampf möglich.

Nach der Umsetzung kann man die entstandenen Aldehyde und Ketone nach üblichen Techniken, z.B. durch Destillation aus dem Reaktionsgemisch isolieren: nicht-umgesetzte Ausgangsstoffe werden gegebenenfalls, wenn nötig, in die Umsetzung zurückgeführt. Auch eine direkte Weiterverarbeitung der Reaktionsprodukte ist aufgrund der sehr hohen Ausbeuten möglich. Bei dem erfindungsgemäßen Verfahren fallen vorzugsweise die monomeren Verbindungen an. Wenn auch Oligomere, z.B. trimere Aldehyde gebildet werden, so kann man diese abtrennen und nach bekannten Methoden zu den gewünschten Monomeren spalten.

Durch Wahl des Katalysators und der Reaktionsbedingungen, insbesondere der Temperatur, kann bei der Umsetzung der Epoxide der Formel (II) das Verhältnis Aldehyd zu Keton beeinflußt werden. Aldehyde lagern sich teilweise in Ketone um, wie in DE-OS 34 19 378 bzw. dehydratisieren zu Dienen wie in DE-OS 34 19 379 beschrieben.

Die nach dem erfindungsgemäßen Verfahren zugänglichen teilweise neuen Verbindungen sind wichtige Zwischenprodukte und können nach geläufigen Methoden auf einfachem Wege zu Aminen, Alkoholen und Säuren, z.B. durch Oxidation mit Sauerstoff oder durch Reduktion z.B. durch katalytische Hydrierung oder aminierende Hydrierung weiterverarbeitet werden.

Beispiele 1 bis 17

Die Reaktionen werden unter isothermen Bedingungen in einem Rohrreaktor (Wendel 0,6 cm, 90 cm Länge) in der Gasphase mindestens 6 Stunden lang durchgeführt. Die Trennung und Charakterisierung der Reaktionsprodukte erfolgt nach üblichen Methoden. Die quantitative Bestimmung der Reaktionsprodukte und der Ausgangsstoffe erfolgt gaschromatographisch und durch CO-Zahl.

Die in den Beispielen verwendeten Katalysatoren sind:

Katalysator A

Ein Aluminosilikatzeolith vom Pentasil-Typ wird unter hydrothermalen Bedingungen bei autogenem Druck und 150 °C aus 650 g hochdispersem $SiO_2$, 203 g $Al_2(SO_4)_3 \cdot 18\,H_2O$ in 10 kg einer wäßrigen 1,6-Hexandiamin-Lösung (Mischung 50:50 Gew.%) in einem Rührautoklaven hergestellt. Nach Abfiltrieren und Auswaschen wird das kristalline Reaktionsprodukt bei 110 °C/124 h getrocknet und bei 500 °C/24 h calciniert. Der Aluminosilikatzeolith enthält 92,8 Gew.% $SiO_2$ und 4,2 Gew.% $Al_2O_3$.

Katalysator A erhält man, indem man den reinen Aluminosilikatzeolith mit einem Verformungshilfsmittel zu 2 mm-Strängen verformt, bei 110 °/16 h trocknet und bei 500 °/124 h calciniert.

Katalysator B

Ein Borosilikatzeolith des Pentasil-Typs wird in einer hydrothermalen Synthese aus 640 g hochdispersem $SiO_2$, 122 g $H_3BO_3$, 8 kg einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und hei 500 °C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit einem Verformungshilfsmittel 2 mm-Stränge hergestellt, die bei 100 °C/16 h trocknet und bei 500 °C/24 h calciniert werden.

Katalysator C

Katalysator C wird aus dem kommerziell erhältlichen Mordenit (Zeolon 900 H®) hergestellt, wobei ein Ionenaustausch mit 20 %iger Ammoniumchloridlösung vorgenommen wird, um den Restnatriumgehalt auf 0,025 Gew.% zu drücken (Na-Wert nach Trocknung bei 110 °C und Calcination bei 500 °C).

Katalysator D

Bei der Herstellung des Katalysators D wird kommerziell erhältliches Gemisch Erionit/Chabazit (Zeolon 500®) mit 20 %iger wäßriger Ammoniumchloridlösung solange ionenausgetauscht, bis das bei 500 °C calcinerte Material einen Restnatriumgehalt von 0,11 Gew.% oder weniger aufweist.

Katalysator E

Kommerziell erhältlicher L-Zeolith (Baylith L®) wird mit Boehmit im Gewichtsverhältnis 80:20 zu 2 mm-Strängen verformt. Nach Trocknen bei 110 °C/16 h und Calcination bei 500 °C/16 h liegt der fertige Katalysator E vor.

Katalysator F

Der Eisensilikatzeolith vom Pentasil-Typ wird unter hydrothermalen Bedingungen bei autogenem Druck und 165 °C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Schwefelsäure und 425 g Wasser in einem Rührautoklaven während 4 Tagen synthetisiert. Der Zeolith wird abfiltriert, ausgewaschen, bei 110 °C/24 h getrocknet und bei 500 °C/24 h calciniert. Man erhält einen Eisensilikatzeolith mit einem $SiO_2/Fe_2O_3$-Verhältnis von 17,7 und einen $Na_2O$-Gehalt von 1,2 Gew.%. Der Katalysator wird zu 2,5 mm-Strängen verstrangt, bei 110 °C/16 h getrocknet und bei 500 °C/24 h calciniert. Diese Stränge werden mit wäßriger $NH_4$-Lösung (20 %ig) nach bekannter Methode behandelt, bis der Na-Gehalt 0,1 Gew.% (nach Calcination bei 500 °C/5 h) beträgt.

Die Versuchsergebnisse sind in den folgenden Tabellen zusammengestellt.

Tabelle 1

| Beispiel | 1 | 2 | 3 | 4 | 5 |
|---|---|---|---|---|---|
| Einsatzstoff | 2-Methyl-2,3-epoxibutan | | | | |
| Katalysator | A | A | A | A | B |
| Temperatur | 150 °C | 200 °C | 250 °C | 300 °C | 300 °C |
| WHSV | 1,8 $h^{-1}$ | 2,4 $h^{-1}$ | 1,8 $h^{-1}$ | 1,8 $h^{-1}$ | 1,8 $h^{-1}$ |
| Umsatz % | 100 | 100 | 100 | 100 | 100 |
| Wertprodukte im Austrag in Gew.-% | | | | | |
| Methylisopropylketon | 51,6 | 47.4 | 51,3 | 72.5 | 77.3 |
| Pivalaldehyd | 40,4 | 39,8 | 34,6 | 8,8 | 4,6 |
| Isopren | 7,4 | 12,4 | 13,5 | 17,3 | 15,8 |

Tabelle 2

| Beispiel | 6 | 7 | 8 |
|---|---|---|---|
| Einsatzstoff | 2-Methylstyroloxid | | |
| Katalysator | A | B | B |
| Temperatur | 300 °C | 250 °C | 300 °C |
| WHSV | 2,5 $h^{-1}$ | 1,7 $h^{-1}$ | 2,5 $h^{-1}$ |
| Umsatz % | 100 | 100 | 100 |
| Wertprodukte im Austrag in Gew.-% | | | |
| 2-Phenylpropanal | 61,1 | 76,0 | 78,8 |
| Phenylaceton | 18,3 | 16,5 | 11,7 |

Tabelle 3

| Beispiel | 9 | 10 | 11 |
|---|---|---|---|
| Einsatzstoff | 1,1-Diphenylethylenoxid[1)] | | |
| Katalysator | A | A | B |
| Temperatur | 300 °C | 350 °C | 300 °C |
| WHSV | 2,0 $h^{-1}$ | 2,2 $h^{-1}$ | 2,2 $h^{-1}$ |
| Umsatz % | 100 | 100 | 100 |
| Wertprodukte im Austrag in Gew.-%[2)] | | | |
| Diphenylacetaldehyd | 76,7 | 69,3 | 98,5 |
| Phenylbenzylketon | 21,5 | 26,5 | 0,7 |

1) 1,1-Diphenylethylenoxid in THF im Gewichtsverhältnis 50 : 50 gelöst
2) THF herausgerechnet

Tabelle 4

| Beispiel | 12 [2)] | 13 [2)] | 14 [2)] | 15 [2)] | 16 [2)] | 17 |
|---|---|---|---|---|---|---|
| Einsatzstoff | Diisobutylenoxid [1)] | | | | | |
| Katalysator | A | B | C | D | E | F |
| Temperatur | 300 °C | 300 °C | 300 °C | 300 °C | 300 °C | 300 °C |
| WHSV | 2,0 $h^{-1}$ | 2,0 $h^{-1}$ | 1,8 $h^{-1}$ | 1,9 $h^{-1}$ | 2,0 $h^{-1}$ | 1,9 $h^{-1}$ |
| Umsatz % | 100 | 100 | 100 | 100 | 100 | 100 |
| Wertprodukt im Austrag in Gew.-% | | | | | | |
| 2,2,4-Trimethylpentanal | 66,5 | 68,8 | 67,5 | 57,2 | 64,8 | 62,7 |

1) Das eingesetzte Diisobutylenoxidgemisch enthält 70,9 Gew.-% 2,2,4-Trimethyl-4,5-epoxipentan und 20,9 Gew.-% 2,2,4-Trimethyl-3,4-epoxipentan

2) Diisobutylenoxidgemisch mit THF im Gewichtsverhältnis 50 : 50 verdünnt.

## Patentansprüche

1. Verfahren zur Herstellung von Aldehyden und Ketonen der Formel (I)

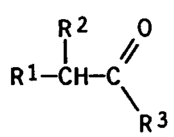

(I)

in der $R^1$ und $R^2$ gerade oder verzweigte Alkylreste mit einer C-Zahl von 1 bis 12, Alkenylreste mit einer C-Zahl von 1 bis 12, Cycloalkyl- oder Cycloalkenylreste mit 5 bis 8 Kohlenstoffatomen, Alkoxyreste oder Aryl- bzw. Aralkylreste, die ihrerseits substituiert sein können, bedeuten und $R^3$ Wasserstoff, Alkyl-, Alkenyl-, Aryl- oder Aralkylreste sein kann, dadurch gekennzeichnet, daß man Epoxide der Formel (II)

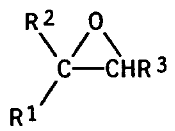

(II)

in der $R^1$, $R^2$ und $R^3$ obige Bedeutung haben, in der Gasphase bei Temperaturen von 150 bis 400 °C an Zeolithen des Pentasil-Typs und/oder des Mordenit-Typs und/oder des Erionit-/Chabazit-Typs und/oder L-Typs katalytisch umlagert.

2. Verfahren nach Anspruch 1, dadurch gekennzeichnet, dap man als Katalysatoren Aluminiumsilikatzeolithe des Pentasil-Typs verwendet.

3. Verfahren nach Anspruch 1, dadurch gekennzeichnet, daß man als Katalysatoren Borosilikatzeolithe des Pentasil-Typs verwendet.

4. Verfahren nach Anspruch 1, dadurch gekennzeichnet, daß man als Katalysatoren Eisensilikatzeolithe des Pentasil-Typs verwendet.

5. Verfahren nach Anspruch 1 bis 4, dadurch gekennzeichnet, daß als Verbindungen der Formel II α-Methylstyroloxid, Diisobutylenoxid, 2-Methyl-2,3-epoxibutan und 1,1-Diphenylethylenoxid eingesetzt werden.

## Claims

1. A process for the preparation of aldehydes and ketones of the formula (I)

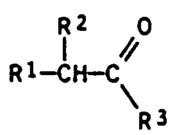

(I)

where $R^1$ and $R^2$ are each straight-chain or branched alkyl of 1 to 12 carbon atoms, alkenyl of 1 to 12 carbon atoms, cycloalkyl or cycloalkenyl, each of 5 to 8 carbon atoms, alkoxy radicals or aryl or aralkyl radicals which in turn may be substituted, and $R^3$ is hydrogen, alkyl, alkenyl, aryl or aralkyl, wherein an epoxide of the formula (II)

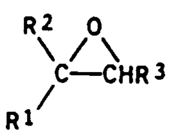

(II)

where $R^1$, $R^2$ and $R^3$ have the above meanings, is subjected to a catalytic rearrangement reaction over zeolites of the pentasil type and/or of the mordenite type and/or of the erionite/chabazite type and/or of the L type in the gas phase at from 150 to 400 °C.

2. A process as claimed in claim 1, wherein the catalyst used is an aluminosilicate zeolite of the pentasil type.

3. A process as claimed in claim 1, wherein the catalyst used is a borosilicate zeolite of the pentasil type.

4. A process as claimed in claim 1, wherein the catalyst used is an iron silicate zeolite of the pentasil type.

5. A process as claimed in any of claims 1 to 5, wherein α-methylstyrene oxide, diisobutylene oxide, 2-methyl-2,3-epoxybutane or 1,1-diphenylethylene oxide is used as the compound of the formula II.

**Revendications**

1. Procédé de préparation d'aldéhydes et de cétones de formule (I)

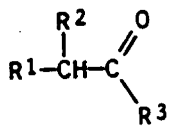

(I)

dans laquelle $R^1$ et $R^2$ représentent des restes alkyle à chaîne droite ou ramifiée ayant un nombre d'atomes de carbone de 1 à 12, des restes alcényle ayant un nombre d'atomes de carbone de 1 à 12, des restes cycloalkyle ou cycloalcényle à 5-8 atomes de carbone, des restes alcoxy ou des restes aryle ou aralkyle, qui peuvent être substitués de leur côté, et $R^3$ peut être un atome d'hydrogène ou un reste alkyle, alcényle, aryle ou aralkyle, caractérisé en ce qu'on transpose par voie catalytique des composés époxy de formule (II)

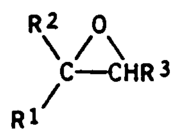

(II)

dans laquelle $R^1$, $R^2$ et $R^3$ ont les significations susindiquées, en phase gazeuse, à des températures de 150 à 400 °C, en présence de zéolithes du type pentasil et/ou du type mordénite et/ou du type érionite/chabazite et/ou du type L.

2. Procédé selon la revendication 1, caractérisé en ce qu'on utilise, comme catalyseurs, des zéolithes d'aluminosilicate du type pentasil.

3. Procédé selon la revendication 1, caractérisé en ce qu'on utilise, comme catalyseurs, des zéolithes de borosilicate du type pentasil.

4. Procédé selon la revendication 1, caractérisé en ce qu'on utilise, comme catalyseurs, des zéolithes de ferrosilicate du type pentasil.

5. Procédé selon l'une quelconque des revendications 1 à 4, caractérisé en ce qu'on utilise, comme composés de formule II, de l'oxyde d'α-méthylstyrène, de l'oxyde de diisobutylène, du 2-méthyl-2,3-époxybutane et de l'oxyde de 1,1-diphényléthylène.